# EUROPEAN PATENT APPLICATION

(11) **EP 4 083 627 A1**
(43) Date of publication of application: **02.11.2022**
(21) Application number: 20904709.1
(22) Date of filing: 25.12.2020
(51) Int. Cl.: G01N 33/574, C07K 14/47, C12Q 1/02

(54) **PROGNOSTIC BIOMARKER OF CANCER**

(30) Priority: 25.12.2019 JP 2019234099
(71) Applicant: Hoshi General Hospital, a Public Interest Incorporated Foundation, Koriyama-shi, Fukushima 963-8501 (JP); Homma, Miwako, Fukushima-shi, Fukushima 960-1295 (JP); Homma, Yoshimi, Fukushima-shi, Fukushima 960-1295 (JP); Hashimoto, Yuko, Fukushima-shi, Fukushima 960-1295 (JP)
(72) Inventor: NOMIZU Tadashi, Koriyama-shi, Fukushima 963-8501 (JP); KIKO Yuichiro, Fukushima-shi, Fukushima 960-1295 (JP); HOMMA Miwako, Fukushima-shi, Fukushima 960-1295 (JP); HOMMA Yoshimi, Fukushima-shi, Fukushima 960-1295 (JP); HASHIMOTO Yuko, Fukushima-shi, Fukushima 960-1295 (JP)
(74) Representative: Mewburn Ellis LLP
(86) International application number: PCT/JP2020/048650
(87) International publication number: WO 2021/132544

(57) **Abstract**

In one embodiment, an object of the present invention is to provide a biomarker for predicting the prognosis of a cancer patient such as a breast cancer patient. In one embodiment, the present invention relates to use of a CK2α protein or a fragment thereof in a nucleolus as a marker for predicting the prognosis of a cancer patient, a method for predicting the prognosis of a cancer patient using the marker, or a kit comprising a reagent for measuring the marker.

## Description

### Technical Field

The present invention relates to a marker for predicting the prognosis of a cancer patient, a method for predicting the prognosis of a cancer patient, and a kit or the like for use in the method.

### Background Art

In Japan, cancer is the primary cause of death among all causes of death, accounting for about 30% of all deaths. For example, breast cancer is the primary cause of death among women aged from 30 to 64 years, and in 2018 the number of deaths due to breast cancer was about 14,000. Although advances in breast cancer detection and/or treatment methods have improved the survival rate of breast cancer patients, there are still some patients with poor prognosis having high risk of relapse, metastasis, or death. Therefore, in order to improve the quality of treatment and/or prevention of breast cancer, it is very important to predict the prognosis of a breast cancer patient, and to individually manage the breast cancer patient according to the result.

Non-Patent Literature 1 reports the relationship between the HER2 gene and protein and the prognosis. However, it cannot be said that the HER2 gene and protein alone can predict breast cancer prognosis with sufficient degree of precision.

### Citation List

### Non-Patent Literature

Non-Patent Literature 1 Ross J.S. et al., Oncologist, 2003, 8(4), pp.307-25.

### Summary of Invention

### Technical Problem

In one embodiment, an object of the present invention is to provide a biomarker for predicting the prognosis of a cancer patient such as a breast cancer patient. In another embodiment, an object of the present invention is to provide a method for predicting the prognosis of a cancer patient such as a breast cancer patient, using the biomarker.

### Solution to Problem

The present inventors found that CK2α protein in a nucleolus can be used as a biomarker for predicting the prognosis of a cancer patient such as a breast cancer patient, and completed the present invention.

The present invention encompasses the following embodiments.
(1) Use of a CK2α protein or a fragment thereof in a nucleolus as a marker for predicting the prognosis of a cancer patient.
(2) The use according to (1), wherein the CK2α protein comprises the amino acid sequence of any of the following (a) to (c):
   (a) the amino acid sequence shown in SEQ ID NO: 2;
   (b) an amino acid sequence in which one or several amino acids are deleted, substituted, or added in the amino acid sequence shown in SEQ ID NO: 2; and
   (c) an amino acid sequence having 90% or more amino acid identity to the amino acid sequence shown in SEQ ID NO: 2.
(3) The use according to (1) or (2), wherein the prognosis comprises relapse risk.
(4) The use according to any one of (1) to (3), wherein said cancer is selected from the group consisting of breast cancer, uterine cancer, esophageal cancer, gastric cancer, biliary tract cancer, pancreatic cancer, liver cancer, renal cancer, colorectal cancer, bladder cancer, lung cancer, thyroid cancer, and glioma.
(5) The use according to (4), wherein said cancer is breast cancer and said marker is combined with at least one of classification by stage, classification by hormone receptor expression status, and classification by HER2 gene and/or protein expression status to predict the prognosis of a breast cancer patient.
(6) A method for predicting the prognosis of a cancer patient, the method comprising:
   a step of detecting a CK2α protein or a fragment thereof in a nucleolus in a cancer cell or a tissue obtained from a cancer patient; and
   a step of predicting a poor prognosis when a CK2α protein or a fragment thereof is detected, and/or predicting a good prognosis when a CK2α protein or a fragment thereof is not detected.
(7) A method for predicting the prognosis of a cancer patient, the method comprising:
   a step of detecting a CK2α protein or a fragment thereof in a nucleolus in a cancer cell or a tissue obtained from a cancer patient; and
   a step of predicting a poor prognosis when a CK2α protein or a fragment thereof is detected in a nucleolus at a higher level compared with other cell fractions, and/or predicting a good prognosis when a CK2α protein or a fragment thereof is not detected in a nucleolus at a higher level compared with other cell fractions.
(8) The method according to (6) or (7), wherein the CK2α protein comprises the amino acid sequence of any of the following (a) to (c):
   (a) the amino acid sequence shown in SEQ ID NO: 2;
   (b) an amino acid sequence in which one or several amino acids are deleted, substituted, or added in the amino acid sequence shown in SEQ ID NO: 2; and
   (c) an amino acid sequence having 90% or more amino acid identity to the amino acid sequence shown in SEQ ID NO: 2.
(9) The method according to any of (6) to (8), wherein the prognosis comprises relapse risk.
(10) The method according to any of (6) to (9), wherein said cancer is selected from the group consisting of breast cancer, uterine cancer, esophageal cancer, gastric cancer, biliary tract cancer, pancreatic cancer, liver cancer, renal cancer, colorectal cancer, bladder cancer, lung cancer, thyroid cancer, and glioma.
(11) The method according to (10), wherein the cancer is breast cancer and whether a CK2α protein or a fragment thereof is detected or not is combined with at least one of classification by stage, classification by hormone receptor expression status, and classification by HER2 gene and/or protein expression status to predict the prognosis of a breast cancer patient.
(12) A kit for use in the method according to any of (6) to (11), comprising a reagent for measuring the amount of a CK2α protein or a fragment thereof.

The present specification encompasses the disclosure of Japanese Patent Application No. 2019-234099, to which the priority of the present application is claimed.

### Advantageous Effects of Invention

The present invention provides a biomarker for predicting the prognosis of a cancer patient such as a breast cancer patient.

### Brief Description of Drawings

[Figure 1] Figure 1 shows representative images for immunohistochemical staining evaluation of a CK2α protein in a breast cancer tissue. Figure 1A is an image (×100) of a cancer invasion area and a normal area next to each other on a single section. Figure 1B is a ×400 image of a cancer invasion area. In Figure 1B, an example of a staining image in which a nucleolus is remarkably positive is indicated by an arrow.
[Figure 2] Figure 2 shows example images of specimens with staining evaluations I-V (I: whole cell is stained, but nucleus staining is not clear; II: nucleus staining (+) with nucleus staining clearer than cytoplasm; III: nucleus staining (++) with nucleus staining higher than II; IV: nucleus staining (+, ++) with nucleolus staining (+); V: nucleus staining (-) with nucleolus staining (+)).
[Figure 3] Figure 3 shows relapse-free survival rate in patients at breast cancer stages I, II, and III.
[Figure 4] Figure 4 shows disease-specific survival rate in patients at breast cancer stages I, II, and III.
[Figure 5] Figure 5 shows relapse-free survival rate in patients at breast cancer stages I-III with three levels of CK2α staining: I+II, III, and IV+V.
[Figure 6] Figure 6 shows relapse-free survival rate in hormone receptor-positive/HER2-negative patients.
[Figure 7] Figure 7 shows relapse-free survival rate in triple negative patients.
[Figure 8] Figure 8 shows relapse-free survival rate in patients at breast cancer stages I and II.
[Figure 9] Figure 9 shows relapse-free survival rate in patient at breast cancer stage III.
[Figure 10] Figure 10 shows relapse-free survival rate in patients with lymph node metastasis.
[Figure 11] Figure 11 shows results of immunohistochemical staining for a CK2α protein in various cancer tissues. Figure 11A is an example of glioma staining (×400). Figure 11B is an example of bladder cancer staining (×400). Figure 11C is an example of renal cancer staining (×400). Figure 11D shows an example of thyroid cancer staining (×400). In Figures 11A to 11D, positive cases of nucleolus are indicated by arrows.
[Figure 12] Figure 12 shows results of immunohistochemical staining for a CK2α protein in various cancer tissues. Figure 12A is an example of pancreatic cancer staining (×400). An example in which one nucleolus was detected in a nucleus and an example in which two nucleoli were detected in a nucleus are shown. Figure 12B is an example of esophageal cancer staining (×400). Figure 12C is an example of biliary tract cancer staining (×400). Figure 12D is an example of uterine cancer staining (×400). In Figures 12A to 12D, positive cases of nucleolus are indicated by arrows.
[Figure 13] Figure 13 shows results of immunohistochemical staining for a CK2α protein in a liver. Figure 13A is an example of healthy human liver staining (×400). Figure 13B is an example of liver cancer staining (×400). Positive cases of nucleolus are indicated by arrows.
[Figure 14] Figure 14 shows results of immunohistochemical staining for a CK2α protein in lung adenocarcinoma and squamous lung cell cancer. Figure 14A is an example of healthy human lung staining (×400). Figure 14B is an example of lung adenocarcinoma staining (×400). Figure 14C is an example of squamous cell lung cancer staining (×400). In Figures 14B and 14C, positive cases of nucleolus are indicated by arrows.
[Figure 15] Figure 15 shows results of immunohistochemical staining for a CK2α protein in a stomach. Figure 15A is an example of healthy human stomach staining (×400). Figure 15B is an example of gastric cancer staining (×400). In Figure 15 and Figure 15B, positive cases of nucleolus are indicated by arrows.
[Figure 16] Figure 16 shows results of immunohistochemical staining for a CK2α protein in a rectum. Figure 16A is an example of rectal cancer-free area staining (×400). Figure 16B is an example of rectal cancer staining (×400). Figure 16C is an example of colon cancer-free area staining (×400). Figure 16D is an example of colon cancer staining (×400). In Figures 16B and 16D, positive cases of nucleolus are indicated by arrows.

### Description of Embodiments

### (Marker)

In one aspect, the present invention relates to a marker for predicting the prognosis of a cancer patient, the marker containing or consisting of a CK2α protein or a fragment thereof in a nucleolus.

As used herein, the type of "cancer" is not limited, and examples thereof include adenocarcinoma, squamous cell carcinoma, small cell carcinoma, and large cell carcinoma. Specific examples of cancer types include malignant melanoma, oral cavity cancer, laryngeal cancer, pharyngeal cancer, thyroid cancer, lung cancer, breast cancer, esophageal cancer, gastric cancer, colorectal cancer (including colon cancer and rectal cancer), small bowel cancer, bladder cancer, prostate cancer, testicular cancer, uterine cancer, cervical cancer, endometrial cancer, ovarian cancer, gastric cancer, renal cancer, liver cancer, pancreatic cancer, biliary tract cancer (including gallbladder cancer and bile duct cancer), brain tumor, head and neck cancer, mesothelioma, osteosarcoma, glioma, a childhood tumor including neuroblastoma, leukemia, and lymphoma. The cancer is preferably breast cancer, uterine cancer, esophageal cancer, gastric cancer, pancreatic cancer, liver cancer, biliary tract cancer (for example, gallbladder or bile duct cancer), renal cancer, colorectal cancer (for example, rectal cancer and colon cancer), bladder cancer, lung cancer (for example, lung adenocarcinoma or squamous cell lung cancer), thyroid cancer, or glioma (for example, astrocytoma), and more preferably breast cancer.

As used herein, the type of "breast cancer" is not limited, and examples thereof include non-invasive breast ductal carcinoma, invasive breast ductal carcinoma, invasive lobular carcinoma, non-invasive lobular carcinoma, and special types of carcinoma such as medullary carcinoma, mucinous carcinoma, or tubular carcinoma.

As used herein, "prognosis" refers to a predicted course (for example, the presence or absence of relapse, or survival or death) in a cancer patient such as a breast cancer patient. "Prediction of prognosis" may be a prediction of relapse risk (for example, relapse-free survival rate), survival time, or survival rate at a certain time after surgery (for example, at the time after 1, 2, 3, 4, 5, 10, 15, or 20 years or longer), relapse-free survival rate (RFS), or disease-free survival rate (DFS). In one embodiment, prediction of prognosis includes prediction of relapse risk (for example, relapse-free survival rate). As used herein, relapse-free survival rate is the proportion of patients free of developing relapsed cancer, such as cancer associated with a first cancer, and disease-specific survival rate is the proportion of patients free of death associated with a first cancer. Prediction of prognosis can also be determination, evaluation, or diagnosis of prognosis, or an assistance thereof.

CK2 (Casein kinase 2) protein is one type of serine/threonine kinase and is known to be involved in a pro-survival pathway, or the like. A CK2-protein is typically present as a tetramer composed of an α subunit, an α' subunit, and two β-subunits. As used herein, "CK2α protein" is intended to refer to the α subunit of CK2, and is also referred to as casein kinase 2 alpha 1 or casein kinase II subunit alpha: CK2α, CK2α1, or CSNK2A1.

Herein, a CK2α protein or a fragment thereof derived from an endogenous gene of a cancer patient, such as a breast cancer patient, can be a biomarker. For example, when the patient is a human, a human CK2α protein or a fragment thereof can be a biomarker.

Specific examples of a CK2α protein include a human-derived CK2α (human CK2α)-protein containing or consisting of the amino acid sequence shown in SEQ ID NO: 2.

The CK2α protein also encompasses a CK2α variant having a functionally comparable activity to the CK2α protein represented by SEQ ID NO: 2, as well as a CK2α orthologue of other species. Specific examples thereof include an amino acid sequence in which one or several amino acids are deleted, substituted, or added in the amino acid sequence shown in SEQ ID NO: 2, or a CK2α protein having 80%, 90%, 95%, 97%, 98%, or 99% or more amino acid identity to the amino acid sequence shown in SEQ ID NO: 2.

As used herein, "several" means, for example, 2-10, 2-7, 2-5, 2-4, or 2-3. As for an amino acid substitution, a conservative amino acid substitution is preferable. A "conservative amino acid substitution" refers to a substitution between amino acids having similar properties such as charge, side chain, polarity, and aromaticity. Amino acids with similar properties can be classified into, for example, a basic amino acid (arginine, lysine, histidine), an acidic amino acid (aspartic acid, glutamic acid), an uncharged polar amino acid (glycine, asparagine, glutamine, serine, threonine, cysteine, tyrosine), a non-polar amino acid (leucine, isoleucine, alanine, valine, proline, phenylalanine, tryptophan, methionine), a branched-chain amino acid (leucine, valine, isoleucine), and an aromatic amino acid (phenylalanine, tyrosine, tryptophan, histidine).

As used herein, "amino acid identity" refers to the proportion (%) of identical amino acid residues between two amino acid sequences relative to the total amino acid residues of a CK2α protein containing the amino acid sequence shown in SEQ ID NO: 2 when two amino acid sequences are aligned and gaps are introduced if necessary to achieve the highest degree of amino acid identity between the two amino acid sequences. Amino acid identity can be calculated using a protein search system by BLAST or FASTA. For details of methods for determining identity, see, for example, Altschul et al, Nuc. Acids. Res. 25, 3389-3402, 1977 and Altschul et al, J. Mol. Biol. 215, 403-410, 1990.

A CK2α protein is encoded by a CK2α gene. Specific examples of the CK2α gene include a human CK2α gene encoding a human CK2α protein containing the amino acid sequence shown in SEQ ID NO: 2. More specific examples of the CK2α include a gene containing or consisting of the base sequence shown in SEQ ID NO: 1.

The CK2α gene encompasses a CK2α gene encoding a CK2α variant having functionally comparable activity to a CK2α protein encoded by the CK2α gene shown in SEQ ID NO: 1, or a CK2α gene encoding a CK2α ortholog of other species. Specifically, the CK2α gene encompasses a CK2α gene having the base sequence shown in SEQ ID NO: 1 in which one or several bases are deleted, substituted, or added, or having 80% or more, 90% or more, 95% or more, 97% or more, 98% or more, or 99% or more base identity to the base sequence shown in SEQ ID NO: 1. Furthermore, the CK2α gene encompasses a gene that contains a base sequence that hybridizes under high-stringent conditions with a nucleic acid fragment containing a portion of a complementary base sequence to the base sequence shown in SEQ ID NO: 1 and that encodes a protein having functionally comparable activity to a CK2α protein.

As used herein, "base identity" refers to the proportion (%) of identical bases between two base sequences relative to the total bases of the CK2α gene containing the sequence shown in SEQ ID NO: 2, when the two base sequences are aligned and gaps are introduced if necessary to achieve the highest degree of base identity between the two.

As used herein, "hybridize under high-stringent conditions" refers to hybridization and washing under low salt concentration and/or high temperature conditions. For example, an incubation is performed with a probe in 6×SSC, 5×Denhardt's reagent, 0.5% SDS, 100 µg/mL denatured fragmented salmon sperm DNA at from 65°C to 68°C, followed by washing in 2×SSC, 0.1% SDS washing solution starting at room temperature, lowering the salt concentration in the washing solution to 0.1×SSC, and raising the temperature to 68°C until no background signal is detected. The conditions for high-stringent hybridization can be found in Green, M.R. and Sambrook, J., 2012, Molecular Cloning: A Laboratory Manual Fourth Ed., Cold Spring Harbor Laboratory Press, Cold Spring Harbor, New York for reference.

The base sequence information of such a CK2α gene can be searched from public databases (GenBank, EMBL, DDBJ). For example, based on the known base sequence information of the CK2α gene shown in SEQ ID NO: 1, genes having high base identity can be searched and obtained from the databases.

As used herein, a "fragment" of a CK2α protein is a peptide fragment containing or consisting of a portion of an amino acid sequence constituting the CK2α protein, which can be identified as a fragment of the CK2α protein from the amino acid sequence constituting the fragment. For example, a "fragment" may be 5 or more, 8 or more, 10 or more, 20 or more, 30 or more, 40 or more, or 50 or more contiguous amino acid residues of the full-length amino acid sequence of a CK2α protein, or a peptide consisting of 200 or less, 150 or less, 120 or less, 100 or less, or 80 or less contiguous amino acid residues. For example, a "fragment" may be a peptide consisting of from 5 to 200, from 10 to 120, or from 50 to 80 contiguous amino acid residues.

As used herein, a "nucleolus" refers to a region of high molecular density in the nucleus of a eukaryotic cell where rRNA transcription and ribosome production take place. A nucleolus is generally observable under a light microscope. Usually, one nucleolus is observed within a nucleus, but a plurality of nucleoli may be observed.

### (Use as a marker for predicting prognosis)

In one aspect, the present invention relates to use of a CK2α protein or a fragment thereof in a nucleolus as a marker for predicting the prognosis of a cancer patient.

In one embodiment, the cancer is selected from the group consisting of breast cancer, uterine cancer, esophageal cancer, gastric cancer, pancreatic cancer, liver cancer, biliary tract cancer, renal cancer, colorectal cancer, bladder cancer, lung cancer, thyroid cancer, and glioma.

In one embodiment, the present invention combines the above-described marker with factors such as classification by stage, tumor diameter, presence of lymph node metastasis, and histological grade to predict the prognosis of a cancer patient.

In one embodiment, in the present invention, the cancer is breast cancer, and the marker is combined with at least one, such as two, preferably all three, of the following: classification by stage, classification by hormone receptor expression status, and classification by a HER2 gene and/or protein expression status to predict the prognosis of a breast cancer patient. In one embodiment, the above-described marker is combined with other factors such as tumor diameter, presence or absence of lymph node metastasis, and histological grade in addition to or separately from the above-described classification, to predict the prognosis of a breast cancer patient. Combination with other classifications or factors can have an effect of enabling more excellent prognosis prediction.

As used herein, classification by stage is a stage classification based on TNM classification (UICC International Code, L.H. Sobin, M.K. Gospodarowicz and Ch. Wittekind, TNM Classification of Malignant Tumours, 7th edition) of the Union for International Cancer Control (UICC). The above-described TNM classification of the Union for International Cancer Control (UICC) is herein referred to as the UICC-TNM classification. In the UICC-TNM classification, breast cancer is classified into stages 0, I, II, III, and IV from the least advanced. The UICC-TNM classification classifies the progression of cancer lesions according to three factors: lump size and spread within a breast (T classification), lymph node metastasis (N classification), and distant metastasis (M classification). Stage determination based on the UICC-TNM classification can be made in accordance with the ordinary knowledge of those skilled in the art.

Specifically, Stage 0 is defined as breast cancer that remains within mammary ducts; Stage I is defined as breast cancer with a tumor diameter of 2 cm or less without axillary lymph node metastasis or with micrometastasis of 0.2 mm or less; Stage II is defined as tumor diameter greater than 2 cm without axillary lymph node metastasis or tumor diameter of 5 cm or less with 3 or less axillary lymph node metastases; and Stage III is defined as 4-9 axillary lymph node metastases regardless of tumor diameter (including clinically evident parasternal lymph node metastases even in the absence of axillary lymph node metastases), or tumor diameter greater than 5 cm with 9 or fewer axillary lymph nodes, or tumor invasion of the chest wall, skin ulcer, skin satellite node, or skin edema regardless of tumor diameter, or inflammatory breast cancer, regardless of lymph node metastases. When there are 10 or more axillary lymph node metastases or axillary lymph node and parasternal lymph node metastases, or ipsilateral supraclavicular lymph node metastases, any tumor status is defined as Stage III. When distant metastasis is present, the tumor is defined as Stage IV. In the following Examples, all cases are shown in terms of the stage after postoperative pathological diagnosis is made (p-stage).

Classification by hormone receptor expression status refers to the expression status of estrogen receptor (ER) and/or progesterone receptor (PgR), such as presence or absence of expression (positive or negative) or high or low expression. The expression status of ER and PgR may be the expression status of genes encoding these proteins, but preferably the expression status of these proteins. Classification by HER2 gene and/or protein expression status may be based on the presence or absence of the HER2 gene and/or protein (positive or negative) or high or low expression of the HER2 gene and/or protein. Methods for measuring the expression status of ER, PgR, and HER2 are known to those skilled in the art and are not limited, and examples of methods for detecting proteins include an immunological detection method such as immunohistochemical staining, and examples of methods for detecting nucleic acids include a nucleic acid amplification method using a primer or a hybridization method using a probe (such as FISH (Fluorescence In Situ Hybridization) method).

When ER, PgR, and HER2 are combined for classification, they can be classified into the following three groups: (1) hormone receptor positive/HER2 negative, in which ER and/or PgR are expressed and HER2 is not expressed; (2) HER2 positive, in which HER2 is expressed regardless of the presence or absence of the expression of ER and PgR; and (3) triple negative, in which neither ER, PgR, nor HER2 is expressed.

In one embodiment, the presence or absence or high or low expression of a CK2α protein or a fragment thereof in a nucleolus is used as a marker for predicting the prognosis of a cancer patient such as a breast cancer patient. The presence or absence or high or low expression thereof is described in detail below.

In one aspect, the present invention relates to a method for predicting the prognosis of a cancer patient. The present method comprises: a step of detecting a CK2α protein or a fragment thereof in a nucleolus in a cancer cell or a tissue obtained from a cancer patient; and a step of predicting a poor prognosis when a CK2α protein or a fragment thereof is detected and/or predicting a good prognosis when a CK2α protein or a fragment thereof is not detected. The detection step can be performed in vitro.

In one aspect, the present invention relates to a method for predicting the prognosis of a cancer patient. The present method comprises a step of detecting a CK2α protein or a fragment thereof in a nucleolus in a cancer cell or a tissue obtained from a cancer patient; and a step of predicting a poor prognosis when a CK2α protein or a fragment thereof is detected in a nucleolus at a higher level compared with other cell fractions, and/or predicting a good prognosis when a CK2α protein or a fragment thereof is not detected in a nucleolus at a higher level compared with other cell fractions. Here, "other cell fractions" are not limited to cell fractions other than the nucleolus, and can be, for example, cytoplasm or nucleoplasm (nucleosol). In addition, "when a CK2α protein or a fragment thereof is not detected in a nucleolus at a higher level compared with other cell fractions" comprises a case where a CK2α protein or a fragment thereof is detected in a nucleolus to the same extent as in other cell fractions (including a case where a CK2α protein or a fragment thereof is detected uniformly throughout the cell) and a case where a CK2α protein or a fragment thereof is detected in other cell fractions at a higher level degree than in the nucleolus. The detection step can be performed in vitro.

Each process is described in detail below.

### (1) Detection step

The stage of a cancer that a patient subject to the present invention suffers from is not limited. For example, in the case of breast cancer, the breast cancer from which a patient subject to the present invention suffers may be breast cancer of stage I-IV, such as stage I-III or stage III.

The cancer patient in the present invention is, for example, a mammal, preferably a primate, and more preferably a human.

A cancer cell or a tissue used in the present invention can be obtained from a cancer patient, for example, by biopsy or resection surgery, although not particularly limited thereto. The cell or tissue may be used as is for detection of a marker, or may be pretreated as appropriate for measurement. For example, paraffin-embedded sections may be prepared from patient-derived samples when the marker is detected by immunohistochemical staining. For example, when the biomarker is detected by Western blotting, a protein extract may be prepared by isolating a nucleus or a nucleolus from a patient-derived sample.

A marker to be detected in the present method may be any of a CK2α protein or a fragment thereof. The detection encompasses measurement of the presence or absence of expression, the amount of expression or the larger or smaller concentration of expression, and the like. As used herein, the term "detection" encompasses any of measurement, qualitative, quantitative and semi-quantitative.

The method for detecting a CK2α protein or a fragment thereof may be any known protein detection method and is not particularly limited, and examples thereof include an immunological detection method.

The "immunological detection method" is a method for measuring the amount of a target molecule using an antibody or antibody fragment that specifically binds to the target molecule which is an antigen.

An antibody can be derived from any animal, including mammals and birds. Examples thereof include a mouse, a rat, a guinea pig, a rabbit, a goat, a donkey, a sheep, a camel, a horse, a chicken, or a human.

An antibody used in an immunological detection method is not particularly limited, and a monoclonal antibody or a polyclonal antibody may be used.

As used herein, "monoclonal antibody" refers to a clonal group of single immunoglobulins. Each immunoglobulin constituting a monoclonal antibody comprises a common framework region and a common complementarity-determining region, and can recognize and bind to the same epitope of the same antigen. A monoclonal antibody can be obtained from a hybridoma derived from a single cell.

As used herein, a "polyclonal antibody" refers to a group of a plurality of immunoglobulins that recognize and bind to different epitopes of the same antigen. A polyclonal antibody can be obtained from a serum of an animal after immunizing the animal with a target molecule as an antigen.

When the antibody is a polyclonal antibody or a monoclonal antibody, known immunoglobulin molecules include each class of IgG, IgM, IgA, IgE, and IgD, and the antibody of the present invention may be of any class, such as IgG.

A method of producing a polyclonal antibody or a hybridoma that produces a monoclonal antibody that recognizes and binds to a CK2α protein may be carried out in accordance with a method known in the field for producing an antibody, using a CK2α protein or a fragment thereof as an antigen. An antibody may also be obtained from a manufacturer.

As used herein, "antibody fragment" means a partial fragment of a polyclonal antibody or a monoclonal antibody, which is a polypeptide chain or a complex thereof having an activity substantially comparable to the antigen-specific binding activity of the antibody. Examples thereof include an antibody portion that encompasses at least one antigen binding site, namely, a polypeptide chain containing at least one set of VL and VH, or a complex thereof. Specific examples thereof include a number of well-characterized antibody fragments, such as those produced by cleaving an immunoglobulin with various peptidases. More specific examples thereof include Fab, F(ab')₂, and Fab'. These antibody fragments all encompass an antigen binding site and have an ability to specifically bind to a target molecule, which is an antigen.

Examples of an immunological detection method include an immunohistochemical staining, an enzyme immunoassay measurement (including ELISA method and EIA method), Western blotting, radioimmunoassay (RIA), immunoprecipitation, or flow cytometry.

For the "immunohistochemical staining method," any known method can be used. For example, a patient-derived sample may be fixed in formalin, embedded in paraffin, thinly sliced into tissue pieces, and attached to a glass slide as a section sample. Immunohistochemical staining may be performed on a section sample using a primary antibody that recognizes a CK2α protein or a fragment thereof and a labeled secondary antibody that recognizes the primary antibody, optionally after antigen retrieval by heat treatment.

In a method in which an expression site cannot be confirmed, such as Western blotting, the expression of a CK2α protein or a fragment thereof in a nucleolus can be confirmed by using a sample obtained by isolating the nucleolus in advance.

Each of the above-described measurement methods is a known technology in the field. Therefore, a specific measurement method can be performed in accordance with a known method. For example, a method described in Green, M.R. and Sambrook, J., 2012 (described above) can be referred to.

### (2) Prediction step

In the present step, the prognosis of a cancer patient is predicted based on a measurement result obtained in the above-described measurement step.

In one embodiment, the present step comprises determining whether the cancer cell or the tissue is positive or negative for the marker from the results obtained in the above-described detection step. When the cancer cell or the tissue is negative for the marker, the prognosis for the cancer patient can be predicted as good. On the other hand, when the cancer cell or the tissue is positive for the marker, the prognosis for the cancer patient can be predicted to be poor.

When using immunohistochemical staining, for example, a case in which one or a plurality of cells or cell clusters are stained can be determined as positive, and a case in which there is no counted stained tumor cells can be determined as negative. Alternatively, a case in which the number of stained tumor cells exceeds a certain proportion (for example, 10%, 15%, or 20%) of the total number of tumor cells may be determined as positive, and a case in which the number of stained tumor cells is not more than the above-described proportion of the total number of tumor cells may be determined as negative. In an immunohistochemical staining method, for example, a section can be classified into the following five stages: I, II, III, IV, and V.
I: there is staining of a whole cell, but nucleus staining is not clear
II: nucleus staining (+) with nucleus staining clearer than cytoplasm
III: nucleus staining (++) with a higher level of nucleus staining than II
IV: nucleus staining (+, ++) with nucleolus staining (+)
V: nucleus staining (-) with nucleolus staining (+)

In the above-described classification, IV and V can be determined to be positive for a CK2α protein or a fragment thereof in a nucleolus.

In one embodiment, the prediction step comprises determining whether the expression level of the marker in the cancer cell or the tissue obtained in the detection step is higher or lower (for example, than a certain threshold). When the expression level of a marker in a cancer cell or a tissue is lower than a certain threshold (for example, statistically significantly lower), the prognosis of a cancer patient can be predicted to be good (for example, relative to a population having an expression level higher than a certain threshold). On the other hand, when the expression level of a biomarker in a cancer cell or a tissue is higher than a certain threshold (for example, statistically significantly higher), the prognosis of a cancer patient can be predicted to be poor (for example, relative to a population having an expression level lower than a certain threshold).

The certain threshold may be a control amount measured in a control sample (a control cell or a tissue, such as a control mammary cell or a mammary tissue). The control sample may be derived from a healthy individual (for example, a healthy human), a benign tumor of a mammary gland, or a breast cancer patient (for example, a stage II breast cancer patient). In the present invention, "healthy individual" refers to a healthy individual of the same species as a subject individual who is not suffering from a cancer.

For example, the expression levels in these individuals or the median value, the average value, the upper level, the lower level, or a value within a certain range in a plurality of individuals can be used as a certain threshold. The threshold can be set as appropriate according to the precision of the prediction, or the like, and can be determined, for example, by ROC (receiver operating characteristic curve) analysis.

As used herein, "statistically significant" refers to a case in which the risk rate (significance level) of the obtained value is small, specifically p < 0.05 (less than 5%), p < 0.01 (less than 1%) or p < 0.001 (less than 0.1%). For the statistical test method, any known test method that can determine the presence or absence of significance may be used as appropriate, and is not particularly limited. For example, a Student t-test, a multiple comparison test, and a log-rank test can be used.

As used herein, "poor prognosis" means that the clinical outcome is poor (unfavorable) (for example, after surgical resection) (for example, high relapse risk or relapse rate of a cancer such as a breast cancer, low relapse-free survival rate, low disease (cancer)-specific survival rate, or low overall survival rate). When the prognosis is poor, the relapse-free survival rate or disease-specific survival rate after 5 years may be 95% or less, 90% or less, 85% or less, 80% or less, 75% or less, or 70% or less. In the present invention, the survival rate means the cumulative survival rate.

As used herein, "good prognosis" means that the clinical outcome is good (favorable). When the prognosis is good, the relapse-free survival rate or survival rate after 5 years from surgical resection of a cancer may be 90% or more, 95% or more, or 100%.

According to the present invention, the prognosis of a cancer patient can be predicted, and based on the results, a treatment strategy (for example, the type, the dosage, and the interval of administration of an anticancer drug) can be determined, or an interval for testing for cancer relapse and metastasis can be determined.

When the present invention predicts that the prognosis of a cancer patient is poor, a drug therapy and/or radiation therapy can be carried out for the patient in order to prevent relapse of the cancer or to improve the prognosis or to improve the survival rate. Therefore, the present invention also provides a method for preventing relapse of a cancer or improving prognosis or improving survival rate, comprising administering at least one of a drug therapy or a radiation therapy to a cancer patient who is predicted to have a poor prognosis by the method of the present invention. Further, when the prognosis of a cancer patient is predicted to be poor, the frequency of testing may be increased in order to detect relapse of a cancer at an earlier stage.

Examples of a drug comprise, but are not limited to, an anticancer agent such as doxorubicin, cyclophosphamide, 5-fluorouracil (5-FU), capecitabine, oxaliplatin, and irinotecan; a hormonal therapeutic agent such as an antiestrogen agent (for example, tamoxifen), an LH-RH agonist formulation (for example, leuplin), an aromatase inhibitor (for example, anastrozole), and a progesterone preparation; and an antibody drug such as a HER2 antibody (for example, trastuzumab). A drug can be used alone or in combination. A drug can be administered through a route such as injection, intravenous administration, or oral administration.

In one embodiment, the method described herein combine the presence or absence of detection of a CK2α protein or a fragment thereof with a factor such as classification by stage, tumor diameter, presence or absence of lymph node metastasis, or histological grade to predict the prognosis of a cancer patient.

In one embodiment, in the method described herein, the cancer is breast cancer, and the presence or absence of detection of a CK2α protein or a fragment thereof is combined with at least one of classification by stage, classification by hormone receptor expression status, and classification by HER2 gene and/or protein expression status to predict the prognosis of a breast cancer patient. Classification by stage, hormone receptor expression status, and HER2 gene and/or protein expression status are described in the section (Use as a marker for predicting prognosis). In one embodiment, the method described herein predicts the prognosis of a breast cancer patient by combining the presence or absence of detection of a CK2α protein or a fragment thereof with another factor such as tumor diameter, presence or absence of lymph node metastasis, or histological grade, in addition to or separately from the above-described classification. Combination with another classification or factor can have an effect of enabling more excellent prediction of prognosis.

### (Kit)

In one embodiment, the present invention also provides a kit for predicting the prognosis of a cancer patient, comprising a reagent for measuring the amount of the above-described marker of the present invention.

Examples of a reagent for measuring the amount of a marker comprise an antibody or an antibody fragment, such as those described above. The kit may further include at least one of a known reagent for immunohistochemical staining, ELISA, Western blotting, or the like, such as a labeling reagent, a buffer, a chromogenic substrate, a secondary antibody, a blocking reagent, an instrument and a control necessary for testing and an instruction manual.

Hereinafter, the present invention will be described more specifically using Examples. However, the technical scope of the present invention is not limited to these Examples. Examples

### Example 1: Immunohistochemical staining of a CK2α protein in breast cancer tissue

### (Material and method)

Formalin-fixed, paraffin-embedded specimens of breast cancer tissue resected from 117 patients with primary breast cancer who underwent radical resection between 2007 and 2014 at Hoshi General Hospital were used. Tumor stage was determined according to the TNM classification of malignant tumors (UICC International Code, L.H. Sobin, M.K. Gospodarowicz and Ch. Wittekind, TNM Classification of Malignant Tumours, 7th edition). This study was approved by the review boards of Hoshi General Hospital and Fukushima Medical University.

A formalin block was sectioned at 4 µm thickness and mounted on a glass plate. An antigen was retrieved by autoclaving at 105°C for 10 min in 10 mM sodium bicarbonate buffer (pH 8.0) using Tissue Tech Prisma 6120 (Sakura Finetech Japan Co., Ltd.) after deparaffinization and rehydration in accordance with an ordinary method. The section was subjected to blocking with goat serum diluted 200-fold in 10 mM phosphate-buffered saline (PBS) containing 1% bovine serum albumin (BSA) for 30 minutes at room temperature. After the section was washed with PBS, reaction was performed overnight at 4°C with a mouse monoclonal anti-CK2α antibody (ab70774, Abcam, UK) (antigen is the full-length protein of CK2α) diluted 1,000-fold in PBS containing BSA and 0.05% Tween^{®} 20. After 16 hours, the section was incubated with Biotin conjugated anti-mouse IgG (BA-9200, Vector Laboratories, US) for 30 minutes at room temperature and then with Vectastain^{™}Elite ABC HRP kit (PK-6102, Vector Laboratories, US) for 30 minutes with an avidin-horse radish peroxidase complex, followed by visualization of the anti-CK2α antibody with Diaminobenzidine (DOJINDO, Japan) under acidic conditions. Serial sections were counterstained with hematoxylin.

An immunohistochemical slide with a CK2α antibody was evaluated by two independent pathologists who did not know the patient information at 5 stages of I, II, III, IV, and V according to the following criteria.
I: whole cell is stained, with nucleus staining unclear
II: nucleus staining (+), with nucleus staining clearer than cytoplasm
III: nucleus staining (++), with nucleus staining at a higher level than II
IV: nucleus staining (+, ++), with nucleolus staining (+)
V: nucleus staining (-), with nucleolus staining (+)

### (Result: Immunohistochemical staining)

As a histochemical finding, a CK2α staining image with a predominant level of intranuclear staining was remarkably observed in cancer invasion areas compared to normal areas. Further, cases were also found in which a nucleolus, which is an intranuclear structure, was densely stained. Since CK2α is expressed in all eukaryotic cells, staining of cell body portion is always observed.

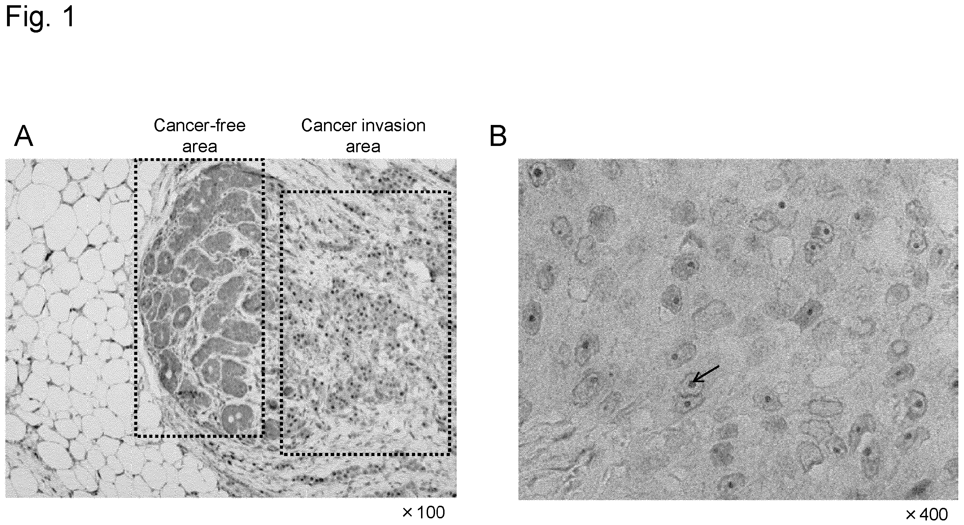

Figure 1 shows representative images for immunohistochemical staining evaluation of a CK2α protein. In Figure 1A, which is an image (×100) of a cancer invasion area and a normal area next to each other on a single section, CK2α protein expression was observed throughout the cell body in normal areas, whereas in cancer-infiltrated areas, cell nuclei were observed more densely than in other cell body areas. Figure 1B is a ×400 image of a cancer invasion area. In a cancer invasion area, a "nucleolus," a structure within a cell nucleus, was observed to be remarkably positive in the staining image (an example is shown by an arrow in Figure 1B).

A similar staining image was observed with another antibody (not commercially available, rabbit polyclonal, the antigen is a peptide consisting of 16 amino acids at the C-terminus of a CK2α). This indicates that any antibody that recognizes CK2α can be widely used for evaluation.

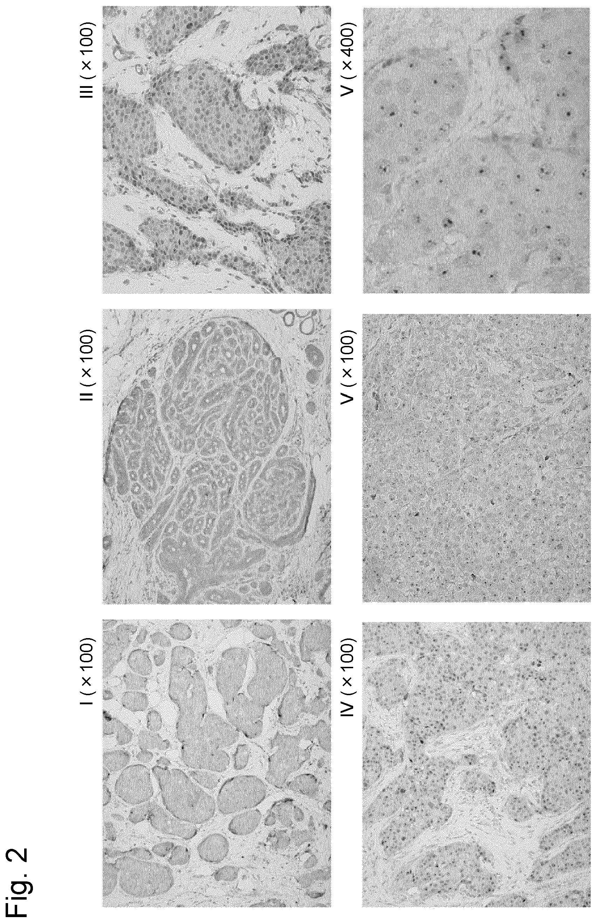

Figure 2 shows exemplary images of specimens with staining evaluations I-V. Of the 117 breast cancer sections, there were 25 cases (21.4%) of nucleolus staining positive IV, and 18 cases (15.4%) of nucleolus staining positive V, and nucleolus staining positive cases accounted for 36.8% of the total. Of the 43 sections that were positive for nucleolar staining, 16 cases were derived from breast cancer patients with stage I, 19 cases from stage II, 7 cases from stage III, and 1 case from stage IV. Additionally, there were 7 cases (6.0%) of staining evaluation I which is nucleolus staining negative, 15 cases (12.8%) of II, and 52 cases (44.4%) of III. Of the 22 sections with staining evaluations I and II, in which nuclear staining levels were low, 13 cases were derived from stage I patients, 8 cases from stage II, and 1 case from stage III.

These results indicated that CK2α protein is present throughout the cell in normal cells, but many cases of high expression in the nucleus are observed in breast cancer cells, that CK2α protein is localized to the nucleolus within the nucleus in some breast cancer patients (a little less than 40%), and that when the nuclear expression level of CK2α protein and nucleolus staining level are higher, the percentage of the cases at higher stages of breast cancer is higher.

### Example 2: Evaluation of prognosis after resection surgery in breast cancer patients

Among the breast cancer patients described in Example 1, the prognosis of 113 primary breast cancer patients at stages I-III excluding stage IV was evaluated. The determination of tumor stage is as described in Example 1. Patient clinical information was obtained retrospectively by reviewing medical records. Patient background is shown in Table 1.

Prognostic events were relapse, breast cancer death, and all-cause death, and the relationship with CK2α staining evaluation was examined.

Furthermore, the survival curves of CK2α nucleolus staining positive (IV + V) and negative (I + II + III) were analyzed by the Kaplan-Meier method. Relapse-free survival, disease-specific survival, and overall survival were analyzed. Relapse-free survival, disease-specific survival, and overall survival are defined as the time from surgery to relapse, the time from the date of surgery to death from breast cancer, and the time from surgery to death from any cause, respectively. Significant differences between the two survival curves for CK2α nucleolus staining positive (IV+V) and negative (I+II+III) were tested by log-rank test, and hazard ratios and their 95% confidence intervals were calculated. All statistical analyses were performed using Graphpad Prism 7.0.

### [Table 1]

**Table 1: Patient background**

| | | N = 113 |
|---|---|---|
| Median age (min to max) | | 55 (26-98) |
| Subtype | Hormone receptor-positive/HER2-negative | 71 (62.8%) |
| | HER2-positive | 23 (20.4%) |
| | Triple negative | 19 (16.8%) |
| Median tumor diameter, cm (min to max) | | 2.0 (0.1-12.0) |
| p-stage | I | 45 |
| | IIA | 35 |
| | IIB | 12 |
| | IIIA | 8 |
| | IIIB | 1 |
| | IIIC | 12 |
| Lymph node metastasis | pN0 | 63 |
| | pN1 | 31 |
| | pN2 | 6 |
| | pN3 | 12 |
| | Unidentified | 1 |
| Histological grade | 1 | 25 |
| | 2 | 54 |
| | 3 | 34 |
| Relapse case | | 12 (10.6%) |
| Fatal case | | 5 (4.4%) |

### (Results)

### (All cases)

Table 2 demonstrates CK2α staining evaluations to which relapse, death from breast cancer, and overall death were attributed. Of 12 cases of relapse, 9 cases were CK2α nucleolus staining positive IV or V, and 3 cases were nuclear strong staining III. Of 5 cases of death due to breast cancer, 4 cases were nucleolus staining positive IV or V and 1 case was nuclear strong staining III.

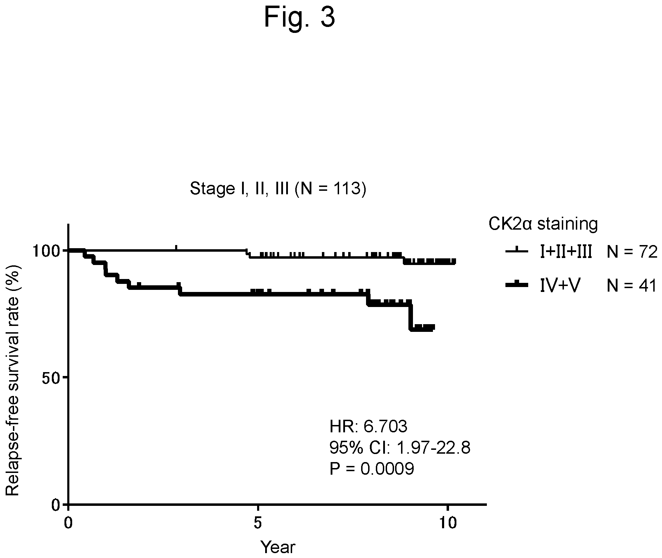

The results of relapse-free survival rate are shown in Figure 3. Breast cancer patients being positive with CK2α nucleolus staining showed a significantly lower relapse-free survival rate compared to patients being negative with nucleolus staining. This indicates that the relapse risk of CK2α nucleolus staining positive cases is significantly higher than that of negative cases.

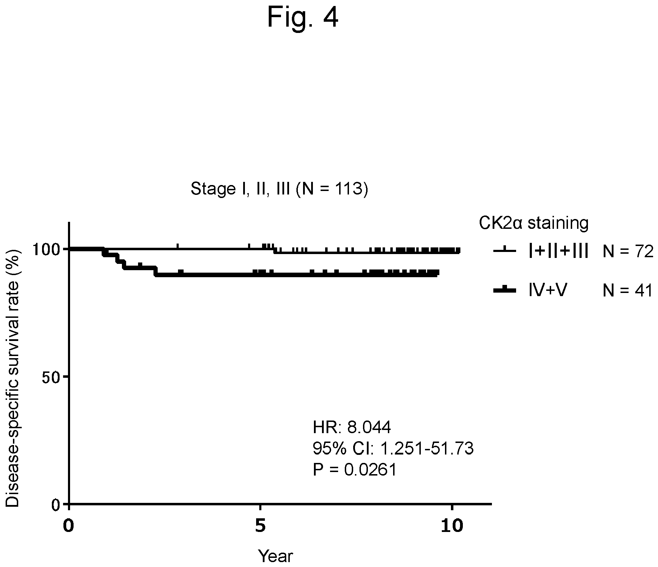

Next, results of disease-specific survival rate are shown in Figure 4. The 10-year survival rate for CK2α nucleolus staining positive cases was 89.8%, significantly lower than 98.5% for negative cases. A trend similar to disease-specific survival rate was observed for overall survival rate (results not shown).

The above demonstrates that nucleolus localization of a CK2α protein represents a high relative risk for both relapse and prognosis of life.

### [Table 2]

**Table 2: Relationships between CK2α staining and prognosis**

| CK2α staining | N | | | |
|---|---|---|---|---|
| | All cases | Relapse | Death from breast cancer | Overall death |
| I+II | 22(19.5%) | 0 | 0 | 0 |
| III | 50(44.2%) | 3 | 1 | 1 |
| IV+V | 41(36.2%) | 9 | 4 | 6 |
| Total | 113 | 12 | 5 | 7 |

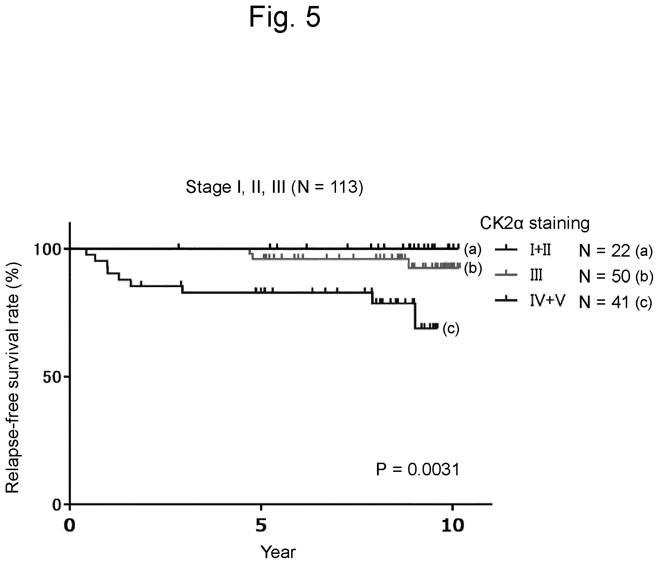

Figure 5 shows results of relapse-free survival rate in breast cancer patients at stages I-III in each case of three levels of CK2α staining: I+II, III, and IV+V. The results indicate that the relapse risk is higher in CK2α nucleolus staining positive cases, also compared to the cases with higher levels of nuclear staining.

### (Subgroup)

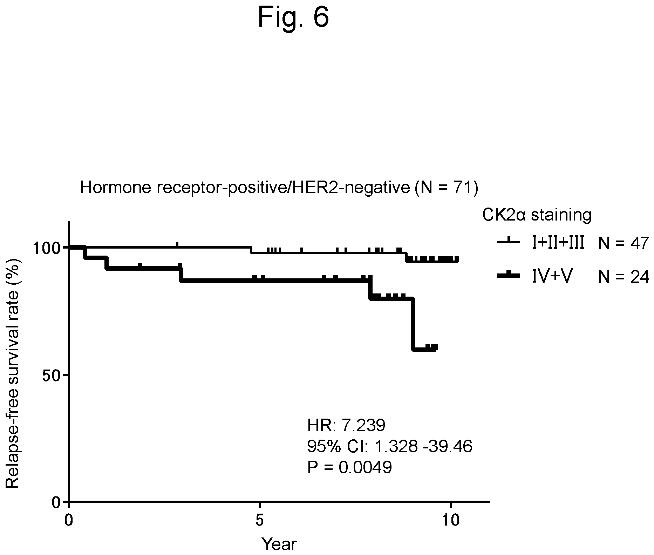

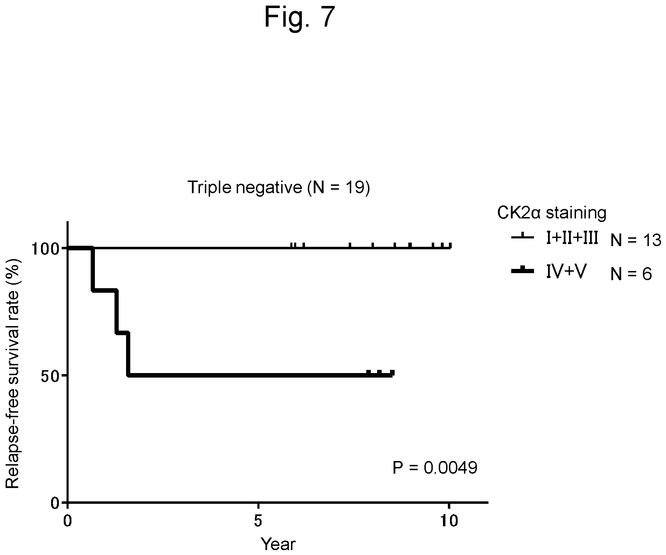

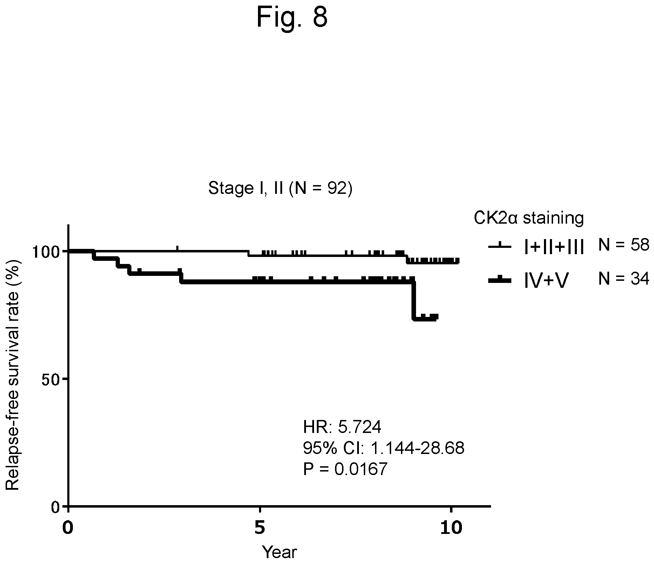

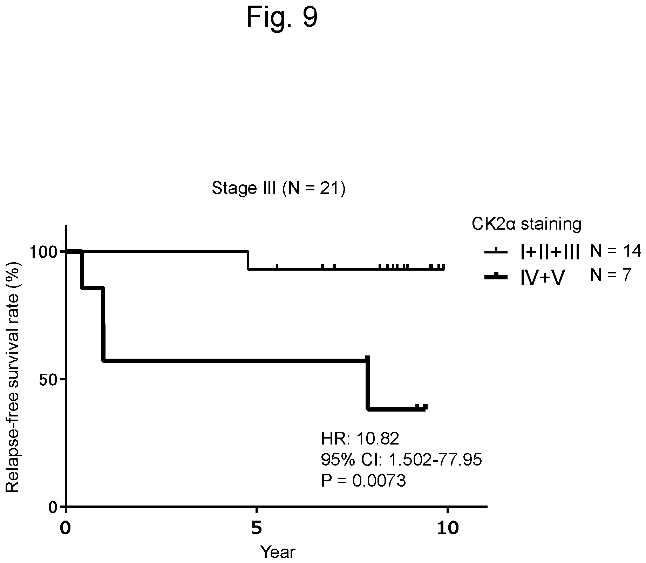

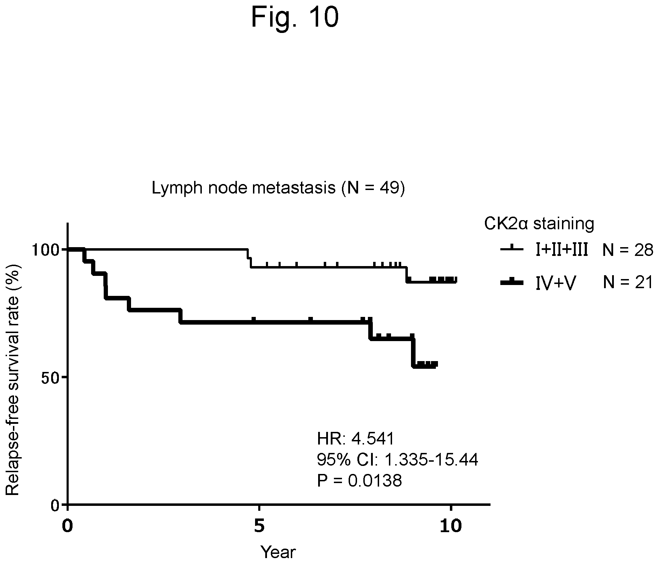

Figure 6 to Figure 10 show results of relapse-free survival rate in subgroups. In any subgroup of hormone receptor-positive/HER2-negative (Figure 6), triple negative (Figure 7), stage I or II (Figure 8), stage III (Figure 9), or with lymph node metastasis (Figure 10), breast cancer patients being positive with CK2α nucleolus staining showed significantly lower relapse-free survival rate compared to nucleolus staining negative patients.

This indicates that the presence or absence of nucleolar localization of a CK2α protein is a relapse predictive factor having predictive power, both in stage I or II or hormone receptor-positive/HER2-negative cases, which are considered to have a relatively good prognosis, and in triple negative, stage III, or lymph node metastatic cases, which are considered to have a relatively poor prognosis. Additionally, since the difference in relapse-free survival between nucleolus-positive and nucleolus-negative for patients with triple negative, stage III, and lymph node metastasis was remarkable, it was suggested that prognosis can be predicted more precisely by combining these factors.

### Example 3: Comparison with other relapse predictive factors

### (Method)

Other relapse predictive factors were analyzed by the Kaplan-Meier method described in Example 2, using information from 113 primary breast cancer patients at stages I-III, as used in Example 2.

### (Results)

Comparison with other relapse predictive factors is shown in Table 3. As shown in Table 3, CK2α nucleolus staining positive (IV + V) had a hazard ratio greater than tumor diameter, stage 3, histologic grade, and triple negative. This indicates that the presence or absence of nucleolar localization of a CK2α protein is a strong relapse predictive factor.

### [Table 3]

**Table 3: Comparison between CK2α nucleolus staining positive and other relapse predictive factors**

| Factor | Hazard ratio | 95% confidence interval | p value |
|---|---|---|---|
| CK2α nucleolus staining-positive | 6.703 | 1.97-22.8 | 0.0009 |
| tumor diameter > 2.0 cm | 4.288 | 1.358-13.54 | 0.0171 |
| Stage 3 | 3.436 | 0.7675-15.38 | 0.0248 |
| Lymph node metastasis | 14.48 | 4.62-45.37 | 0.0007 |
| Hormone receptor-negative | 2.331 | 0.6132-8.861 | 0.1364 |
| Triple negative | 1.804 | 0.3793-8.584 | 0.3688 |
| Histological grade 3 | 1.695 | 0.491-5.85 | 0.3619 |

### Example 4: Immunohistochemical staining of CK2α protein in various cancer tissues

### (Purpose)

Immunohistochemical staining for CK2α protein is performed on various cancer tissues other than breast cancer to examine localization of CK2α protein.

### (Method and result)

### (1) Immunohistochemical staining for CK2α protein in glioma, bladder cancer, renal cancer, thyroid cancer, pancreatic cancer, esophageal cancer, biliary tract cancer, and uterine cancer

Multiple organ cancer tissue array (US Biomax, Inc., product number BC000111b) was used to perform immunohistochemical staining on formalin-fixed, paraffin-embedded specimens derived from various cancer tissues (glioma, bladder cancer, renal cancer, thyroid cancer, pancreatic cancer, esophageal cancer, biliary tract cancer, and uterine cancer). Immunohistochemical staining for CK2α protein was performed in the same manner as in Example 1, using anti-CK2α antibody (ab70774, Abcam, UK).

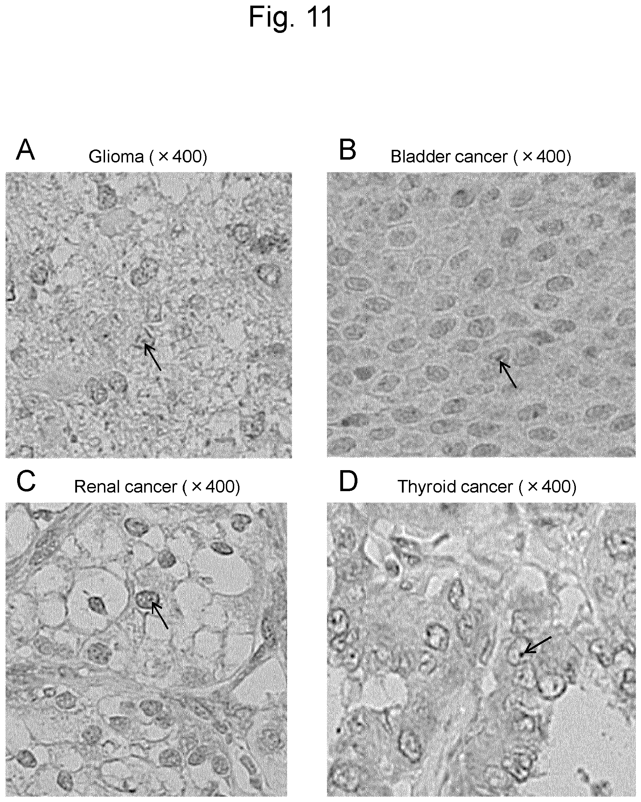

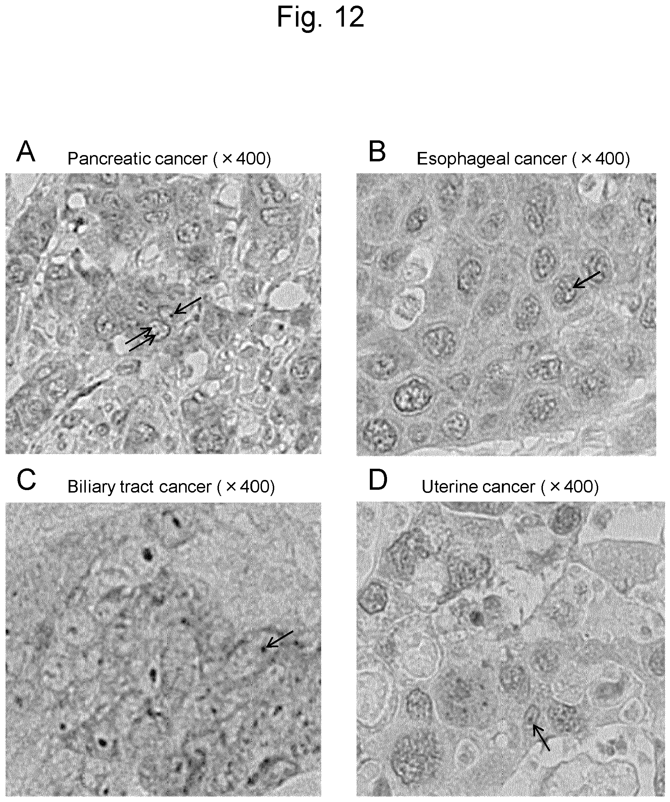

The results of immunohistochemical staining are shown in Figure 11 to Figure 12. A staining image showing positive nucleoli was observed in any of glioma (Figure 11A), bladder cancer (Figure 11B), renal cancer (Figure 11C), thyroid cancer (Figure 11D), pancreatic cancer (Figure 12A), esophageal cancer (Figure 12B), biliary tract cancer (Figure 12C), and uterine cancer (Figure 12D). Examples of nucleolus staining are indicated by arrows in Figure 11 to Figure 12. In some cases, in addition to a nucleolus, a nuclear membrane was also positive.

### (2) Immunohistochemical staining for CK2α protein in liver cancer, lung adenocarcinoma, squamous lung cell cancer, gastric cancer, rectal cancer, and colon cancer

Formalin-fixed, paraffin-embedded specimens of cancer tissue removed from patients with primary cancer (liver cancer, gastric cancer, lung adenocarcinoma, rectal cancer, and colon cancer) who underwent radical resection at Hoshi General Hospital and from patients with primary cancer (squamous cell lung cancer) who underwent radical resection at the Department of Respiratory Surgery, Fukushima Medical University were used. For liver specimens from healthy humans, an FDA standard tissue array (BioChain Institute Inc., product number T8234701-1) was used. Immunohistochemical staining of a CK2α protein was performed in the same manner as in Example 1, using an anti-CK2α antibody (ab70774, Abcam, UK).

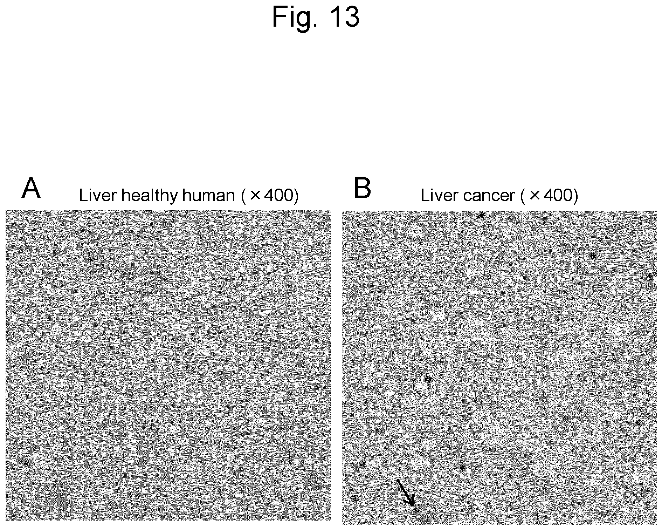

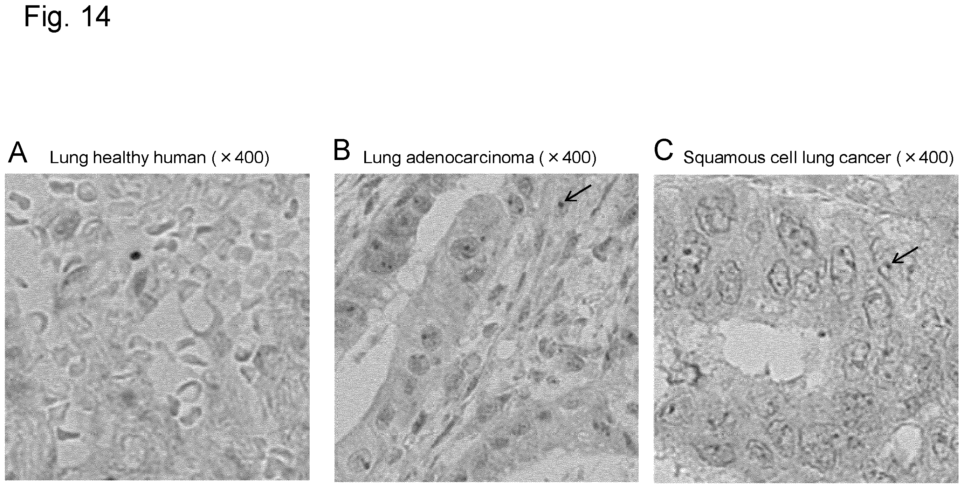

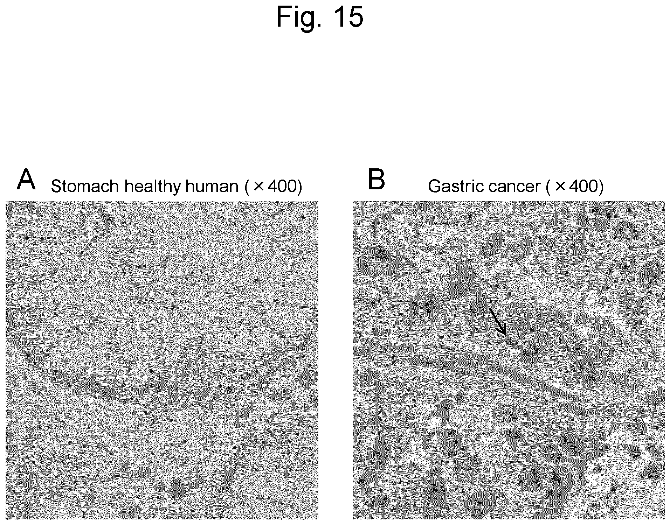

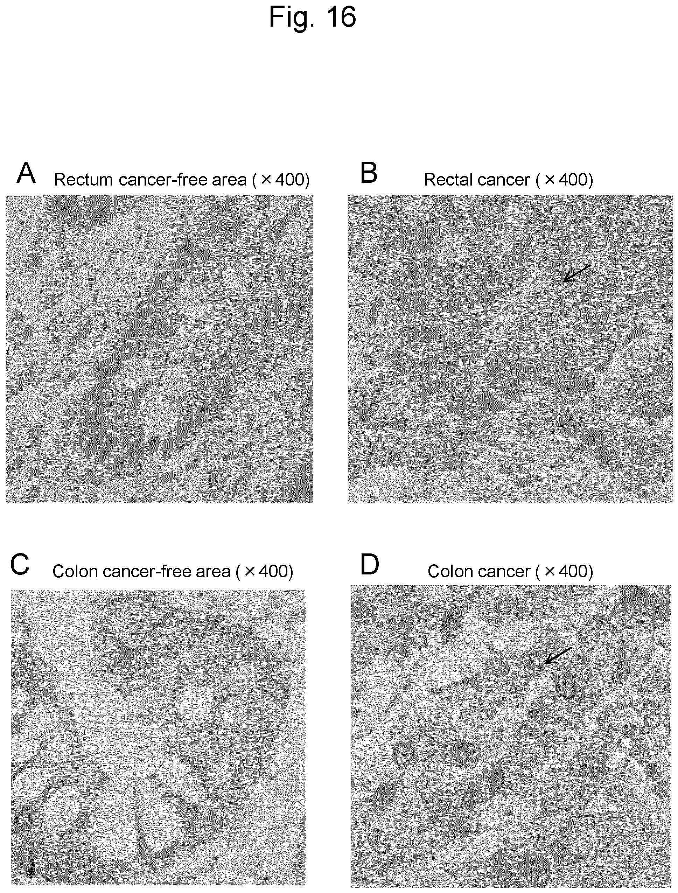

The results of immunohistochemical staining are shown in Figure 13 to Figure 16. A staining image showing positive nucleoli was observed in any of liver cancer (Figure 13B), lung adenocarcinoma (Figure 14B), squamous cell lung cancer (Figure 14C), gastric cancer (Figure 15B), rectal cancer (Figure 16B), and colon cancer (Figure 16D). Examples of nucleolus staining are indicated by arrows in Figure 13 to Figure 16. In some cases, in addition to a nucleolus, a nuclear membrane was also positive.

From the results of (1) and (2) above, it was shown that a CK2α protein can localize to nucleoli in cancers in general, including breast cancer, uterine cancer, esophageal cancer, gastric cancer, biliary tract cancer, pancreatic cancer, liver cancer, renal cancer, colorectal cancer (rectal cancer and colon cancer), bladder cancer, lung cancer (lung adenocarcinoma and squamous cell lung cancer), thyroid cancer, and glioma.

All publications, patents and patent applications cited herein are directly incorporated

## Claims

1. Use of a CK2α protein or a fragment thereof in a nucleolus as a marker for predicting the prognosis of a cancer patient.

2. The use according to claim 1, wherein the CK2α protein comprises the amino acid sequence of any of the following (a) to (c):
(a) the amino acid sequence shown in SEQ ID NO: 2;
(b) an amino acid sequence in which one or several amino acids are deleted, substituted, or added in the amino acid sequence shown in SEQ ID NO: 2; and
(c) an amino acid sequence having 90% or more amino acid identity to the amino acid sequence shown in SEQ ID NO: 2.

3. The use according to claim 1 or 2, wherein the prognosis comprises relapse risk.

4. The use according to any one of claims 1 to 3, wherein said cancer is selected from the group consisting of breast cancer, uterine cancer, esophageal cancer, gastric cancer, biliary tract cancer, pancreatic cancer, liver cancer, renal cancer, colorectal cancer, bladder cancer, lung cancer, thyroid cancer, and glioma.

5. The use according to claim 4, wherein said cancer is breast cancer and said marker is combined with at least one of classification by stage, classification by hormone receptor expression status, and classification by HER2 gene and/or protein expression status to predict the prognosis of a breast cancer patient.

6. A method for predicting the prognosis of a cancer patient, the method comprising:
a step of detecting a CK2α protein or a fragment thereof in a nucleolus in a cancer cell or a tissue obtained from a cancer patient; and
a step of predicting a poor prognosis when a CK2α protein or a fragment thereof is detected in a nucleolus at a higher level compared with other cell fractions, and/or predicting a good prognosis when a CK2α protein or a fragment thereof is not detected in a nucleolus at a higher level compared with other cell fractions.

7. The method according to claim 6, wherein the CK2α protein comprises the amino acid sequence of any of the following (a) to (c):
(a) the amino acid sequence shown in SEQ ID NO: 2;
(b) an amino acid sequence in which one or several amino acids are deleted, substituted, or added in the amino acid sequence shown in SEQ ID NO: 2; and
(c) an amino acid sequence having 90% or more amino acid identity to the amino acid sequence shown in SEQ ID NO: 2.

8. The method according to claim 6 or 7, wherein the prognosis comprises relapse risk.

9. The method according to any one of claims 6 to 8, wherein said cancer is selected from the group consisting of breast cancer, uterine cancer, esophageal cancer, gastric cancer, biliary tract cancer, pancreatic cancer, liver cancer, renal cancer, colorectal cancer, bladder cancer, lung cancer, thyroid cancer, and glioma.

10. The method according to claim 9, wherein said cancer is breast cancer and whether a CK2α protein or a fragment thereof is detected or not is combined with at least one of classification by stage, classification by hormone receptor expression status, and classification by HER2 gene and/or protein expression status to predict the prognosis of a breast cancer patient.

11. A kit for use in the method according to any one of claims 6 to 10, comprising a reagent for measuring the amount of a CK2α protein or a fragment thereof.
